# EUROPÄISCHE PATENTSCHRIFT

(11) **EP 1 875 890 B1**
(45) Veröffentlichungstag und Bekanntmachung des Hinweises auf die Patenterteilung: **30.09.2015**
(21) Anmeldenummer: 07110596.9
(22) Anmeldetag: 19.06.2007
(51) Int. Cl.: A61K 8/35, A61K 8/81, A61Q 5/02, A61Q 5/12, A61Q 5/00, A61K 8/63, A61Q 17/04

(54) **Pflegendes Haarshampoo, das messbar die Farbbrillianz und Farbfrische von gefärbten Keratinfasern verlängert**
Nutritious hair shampoo which demonstrably prolongs the colour brilliance and freshness of dyed keratin fibres
Shampoing capillaire soignant qui prolonge de manière mesurable la brillance et la fraîcheur de la couleur de fibres de kératine colorées

(30) Priorität: 28.06.2006 DE 102006030090
(43) Veröffentlichungstag der Anmeldung: 09.01.2008
(73) Patentinhaber: Beiersdorf Aktiengesellschaft, 20253 Hamburg (DE)
(72) Erfinder: Rieckmann, Olaf, 21244 Buchholz (DE); Albrecht, Harald, 8320 Fehraltorf (CH); Neuhoff, Henrike, 22359 Hamburg (DE)
(74) Vertreter: Hartmann, Jost

(56) Entgegenhaltungen:
- EP-A- 0 808 624
- WO-A-99/55295
- WO-A-2004/045567
- US-A- 5 045 307
- US-A1- 2003 091 518
- US-B1- 6 855 312

## Beschreibung

Zu den wichtigsten Haarbehandlungsmitteln zählen Haarfarbstoffe. Haarfarben, insbesondere künstliche Haarfarben, spielen eine immer wichtigere Rolle in der Haarkosmetik. Die natürliche Haarfarbe kann durch Färbungen mit chemischen und pflanzlichen Farbstoffen verändert werden. Bei der chemischen Haarfärbung wird generell zwischen permanenten und temporären Färbungen unterschieden. Während die permanenten Färbungen den Haaren eine dauerhafte Farbe verleihen sollen, werden Tönungen im wesentlichen zur temporären also vorübergehenden Haarfarbenveränderung benutzt.

Temporäre Colorationen werden üblicherweise nach 6 bis 24 Haarwäschen wieder ausgespült. Durch jede Haarwäsche verändern sich der Farbton, die Farbbrillianz und Farbfrische des gefärbten Haares. Umwelteinflüsse wie z. B. UV-Strahlen, freie Radikale in der Luft etc. führen ebenfalls zu Farbveränderungen. Diese Einflussfaktoren tragen auch bei permanenten Haarfarbstoffen stark zu Farbveränderungen bei.

Der Verbraucher würde gerne so wenig wie möglich Farbveränderungen haben und begrüßt entsprechend eine längere Farbhaftung und Farbbrillianz seiner Coloration. Daher sind kosmetische Reinigungsmittel, die speziell zum Farbschutz/-erhalt gefärbter Haare betragen für den Verbraucher von großem Interesse.

Eine Aufgabe dieser Erfindung bestand daher darin, ein Haarreinigungsmittel zu entwickeln, das zum einen für eine bessere Haftung der Haarfarbstoffe auf den Keratinfasern sorgt und somit zum Farberhalt und Farbschutz beiträgt und zum anderen das gefärbte Haar vor äußeren Umwelteinflüssen schützt. Des weiteren sollte dieses spezielle Haarreinigungsmittel natürlich die Reinigung und insbesondere die Pflege der Haare sicherstellen, denn getöntes und coloriertes Haar benötigt besondere, intensive Pflege, da es durch den Färbevorgang oft stark strapaziert und geschädigt wird.

WO 99/55295 offenbart eine kosmetische Behandlungsmethode zur Verringerung des Ausbleichens des Haares, in der eine Zubereitung mit Conditionierern und UV-Filtern verwendet wird.

EP 1 200 045 offenbart die Verwendung bestimmter Polymere zur Verbesserung der Waschechtheit von Fasern.

DE 103 56 255 offenbart die Verwendung bestimmter Polymere zur Erhaltung der Farbe von gefärbten Keratinfasern.

DE 197 35 865 offenbart die Verwendung bestimmter Polymere und u.a. Grünteeextrakt zur Erhaltung der Farbstabilität von gefärbten Haaren.

EP 0 509 922 offenbart die Verwendung bestimmter Polyorganosiloxanthiole zum Schutz der Farbe gefärbter Haare.

EP 0 940 404 offenbart die Verwendung bestimmter Polyorganosiloxane mit einer antioxidativ wirkenden Gruppe zum Schutz der Farbe gefärbter Haare.

Weiteren Stand der Technik stellen die Schriften WO 94/07455, EP 1 321 131, DE 197 58 271 dar.

Erfindungsgemäße Zubereitungen offenbart keine dieser Schriften und diese weisen auch keinen Weg zur Erfindung.

Überraschenderweise wurde festgestellt, dass eine Haarreinigungszubereitung enthaltend Lichtfilter, nichtionische Filmbildner und Antioxidantien, die einen Delta-E-Wert von weniger als 0,5 erzielt, dadurch gekennzeichnet, dass das Antioxydans Oryzanol ist, den Nachteilen des Standes der Technik abhilft.

Dabei ist es von Vorteil, wenn der Lichtfilter p-Benzophenon ist, bevorzugt in einer Einsatzkonzentration von 0.1 - 0,5 Gew.-%. Dabei ist es von Vorteil, wenn der nichtionische Filmbildner PVP ist, bevorzugt in einer Einsatzkonzentration von 0.1 - 3 Gew-%. Dabei ist es von Vorteil, wenn das Antioxydans Oryzanol in einer Einsatzkonzentration von 0.01 - 1 Gew.-% enthalten ist.

Gegenstand der hier vorliegenden Erfindung ist insbesondere die Verwendung von nichtionischen Filmbildnern, insbesondere von Polyvinylpyrrolidon (PVP), ganz besonders von PVP, welches unter dem Handelsnamen Luviskol K 30 Pulver (BASF) angeboten wird, in Kombination mit Benzophenon-4 und Oryzanol in kosmetischen Haarbehandlungsmitteln zum Schutz der Farbe von Haaren. Ganz besonders vorteilhaft ist es, PVP in Konzentrationen von 0.1 - 3 Gew.-%, Benzophenon-4 von 0.1 - 0.5 Gew.-% und Oryzanol von 0.01 - 1 Gew.-% einzusetzen.

Dabei ist es von Vorteil, wenn zusätzlich ein Tensid enthalten ist.

Als Tenside können anionische, amphotere und nichtionische Tenside in Gesamtkonzentrationen von 2 bis 25 Gew.-% eingesetzt werden. Besonders vorteilhaft ist es, eine Kombination von Laurylethersulfat mit Cocamidopropylbetain einzusetzen. Ganz besonders vorteilhaft ist es, Laurylethersulfat in Konzentrationen von 5 - 15 Gew.-%. und Cocamidopropylbetain in Konzentration von 0.5 - 10 Gew.-% einzusetzen. Als ebenso sehr vorteilhaft hat sich die Kombination von Laurylethersulfat mit Cocamidopropylbetain und Decyl Glucosid erwiesen mit Konzentrationen von 2 - 12 Gew.-% Laurylethersulfat, 2 - 10 Gew.-% Cocamidopropylbetain und 2 - 5 Gew.-% Decyl Glucosid. Besonders bevorzugt ist es, wenn als Tensid die Kombination von Laurylethersulfat mit Cocamidopropylbetain und Sulfosuccinat verwendet wird.

Dabei ist es von Vorteil, wenn zusätzlich ein Conditioner enthalten ist.

Zur Pflege des Haares sind in dieser speziellen Zubereitung besonders vorteilhaft die Kombination der üblichen polymeren Haarkonditioniermittel (Polyquaternium-10, Guar Quats, Polyquaternium-7).

Dabei ist es von Vorteil, wenn das Massenverhältnis von Conditioner zu Tensid zwischen 0,001 und 1 gewählt wird.

Als Verdicker werden bevorzugt Natriumchlorid, PEG-200 hydrogeniertes Glycerylpalmitat und Acrylat Copolymere verwendet. Besonders vorteilhaft sind Einsatzkonzentrationen von 0.05 - 5 Gew.-%.

Als Perlglanzsysteme können prinzipiell alle gängigen, bekannten Perlglanzrohstoffe verwendet werden. Besonders vorteilhaft ist die Verwendung von PEG-3 Distearat. Ganz besonders vorteilhaft ist es, wenn dieser Perlglanzrohstoff eine Teilchengröße aufweist in der Art, dass 90 % der Volumenanteile eine Teilchengröße von kleiner 50µm aufweisen. Die Einsatzkonzentrationen des Perlglanzes liegen vorteilhafterweise bei 5 - 15 Gew.-%.

### Beispiele

| | **1** | **2** | **3** | **4** | **5** | **6** | **7** |
|---|---|---|---|---|---|---|---|
| | | | | | | | |
| Natrium Laurylethersulfat | 9 | 8 | 9 | 9 | 9 | 5 | 9 |
| Cocamidopropyl Betain | 4 | 5 | 4 | 4 | 2 | 5 | 3 |
| Di-Natrium PEG-5 Laurylcitrat Sulfosuccinat | 3 | 0 | 3 | 2 | 5 | 5 | 3 |
| Decyl Glucoside | - | - | - | - | - | 3 | - |
| PEG-200 Hydrogenated Glyceryl Palmate | | - | - | - | - | 0,1 | 0,5 |
| Polyquaternium-7 | 0,3 | 0,3 | - | - | - | - | - |
| Polyquaternium-10 | - | 0,2 | 0,3 | 0,2 | 0,1 | 0,2 | 0,3 |
| Guar Hydroxypropyl-Trimonium Chlorid | 0,1 | - | 0,1 | 0,1 | - | - | 0,1 |
| PVP | 0,25 | 0,3 | 0,5 | 0,5 | 0,5 | 0,5 | 1 |
| Benzophenon-4 | 0,2 | 0,3 | 0,2 | 0,5 | 0,05 | 0,05 | 0,2 |
| Oryzanol | 0,01 | 0,1 | 0,3 | 0,5 | 1 | 1 | 0,05 |
| PEG-3 Distearat | 1,5 | 2 | 2 | 1,5 | 1,5 | 1,5 | 0,7 |
| Glycol Distearat | - | - | - | 0,1 | 0,5 | 0,5 | - |
| PEG-40 hydriertes Rizinusöl | 0,2 | 0,1 | 0,2 | 0,2 | 0,2 | 0,2 | 0,2 |
| Acrylat Copolymer | - | - | - | - | - | - | 0,3 |
| Niacinamid | - | 0,1 | - | - | - | - | - |
| Panthenol | - | 0,1 | - | - | - | - | 0,1 |
| Vitamin E-Acetat | - | - | 0,1 | - | - | - | 0,01 |
| Bambusextrakt | - | - | - | 0,1 | - | - | - |
| Calciumchlorid | - | 0,05 | - | - | - | - | - |
| Aloe Vera | - | - | - | - | 0,1 | 0,1 | - |
| Natrium Salicylat | 0,4 | 0,4 | 0,4 | 0,4 | 0,4 | 0,4 | 0,4 |
| Natrium Benzoat | 0,5 | 0,4 | 0,4 | 0,4 | 0,4 | 0,4 | 0,4 |
| Natriumchlorid | 0,9 | 1,0 | 0,2 | - | - | - | 0,2 |
| Zitronensäure | q.s. | q.s. | q.s. | q.s. | q.s. | q.s. | q.s. |
| Parfüm | q.s. | q.s. | q.s. | q.s. | q.s. | q.s. | q.s. |
| Wasser | ad 100 | ad 100 | ad 100 | ad 100 | ad 100 | ad 100 | ad 100 |
| Delta-E-Wert | 0,5 | 0,5 | 0,35 | 0,36 | 0,46 | 0,45 | 0,37 |
| | | | | | | | |

### Ergebnisse

Natürliche, braune Haarsträhnen wurden mit Wella Koleston 3/0 (dunkelbraun) coloriert. Die Farbe der Strähnen wurde mit dem X-Rite Spectrophotometer SP62 (Machine-No. 126189) entsprechend dem LAB-Farbsystem vermessen. Spektralphotometer arbeitet im Lab-Farbraum mit einer D65 Leuchtquelle (6500 K Farbtemperatur). Die Meßgeometrie entspricht dem 10°-Beobachter.
Anschließend wurden die Strähnen mit den unten aufgeführten Shampoos gewaschen, luftgetrocknet und wieder vermessen.

Der ΔE-Wert beschreibt die gesamte Farbveränderung der Haarsträhnen. Er setzt sich aus der Helligkeit (ΔL), dem rot-grün Anteil (Δa) und dem gelb-blau Anteil (Δb) nach der Formel: ΔE = √ (ΔL² + Δa² + Δb²) zusammen.

## Patentansprüche

1. Haarreinigungszubereitung enthaltend Lichtfilter, nichtionische Filmbildner und Antioxidantien, die einen Delta-E-Wert von weniger als 0,5 erzielt, **dadurch gekennzeichnet, dass** das Antioxydans Oryzanol ist.

2. Zubereitung nach Patentanspruch 1 **dadurch gekennzeichnet, dass** der Lichtfilter p-Benzophenon ist, bevorzugt in einer Einsatzkonzentration von 0.1 - 0.5 Gew.-%.

3. Zubereitung nach einem der vorangehenden Patentansprüche **dadurch gekennzeichnet, dass** der nichtionische Filmbildner PVP ist, bevorzugt in einer Einsatzkonzentration von 0.1 - 3 Gew.-%.

4. Zubereitung nach einem der vorangehenden Patentansprüche **dadurch gekennzeichnet, dass** das Oryzanol in einer Einsatzkonzentration von 0.01 - 1 Gew.-% enthalten ist.

5. Zubereitung nach einem der vorangehenden Patentansprüche **dadurch gekennzeichnet, dass** zusätzlich ein Conditioner enthalten ist.

6. Zubereitung nach einem der vorangehenden Patentansprüche **dadurch gekennzeichnet, dass** als Conditioner Polyquaternium-10, Polyquaternium-7 oder Guar Quats verwendet werden.

7. Zubereitung nach einem der vorangehenden Patentansprüche **dadurch gekennzeichnet, dass** zusätzlich ein Tensid enthalten ist.

8. Zubereitung nach einem der vorangehenden Patentansprüche **dadurch gekennzeichnet, dass** als Tensid die Kombination von Laurylethersulfat mit Cocamidopropylbetain und Sulfosuccinat verwendet wird.

9. Zubereitung nach einem der vorangehenden Patentansprüche **dadurch gekennzeichnet, dass** als Tensid die Kombination von Laurylethersulfat mit Cocamidopropylbetain und Decyl Glucosid verwendet wird.

10. Zubereitung nach einem der vorangehenden Patentansprüche **dadurch gekennzeichnet, dass** das Massenverhältnis von Conditioner zu Tensid zwischen 0,001 und 1 gewählt wird.

## Claims

1. Hair cleansing preparation comprising light filters, nonionic film formers and antioxidants which attains a delta E value of less than 0.5, **characterized in that** the antioxidant is oryzanol.

2. Preparation according to Patent Claim 1, **characterized in that** the light filter is p-benzophenone, preferably in a use concentration of 0.1-0.5% by weight.

3. Preparation according to one of the preceding patent claims, **characterized in that** the nonionic film former is PVP, preferably in a use concentration of 0.1-3% by weight

4. Preparation according to one of the preceding patent claims, **characterized in that** the oryzanol is present in a use concentration of 0.01-1% by weight.

5. Preparation according to one of the preceding patent claims, **characterized in that** a conditioner is additionally present.

6. Preparation according to one of the preceding patent claims, **characterized in that** polyquaternium-10, polyquaternium-7 or guar quats are used as conditioner.

7. Preparation according to one of the preceding patent claims, **characterized in that** a surfactant is additionally present.

8. Preparation according to one of the preceding patent claims, **characterized in that** the combination of lauryl ether sulphate with cocamidopropylbetaine and sulphosuccinate is used as surfactant.

9. Preparation according to one of the preceding patent claims, **characterized in that** the combination of lauryl ether sulphate with cocamidopropylbetaine and decyl glucoside is used as surfactant.

10. Preparation according to one of the preceding patent claims, **characterized in that** the mass ratio of conditioner to surfactant is selected between 0.001 and 1.

## Revendications

1. Préparation de nettoyage capillaire contenant un filtre optique, des agents filmogènes non ioniques et des antioxydants, qui atteint une valeur delta E de moins de 0,5, **caractérisée en ce que** l'antioxydant est l'oryzanol.

2. Préparation selon la revendication 1, **caractérisée en ce que** le filtre optique est la p-benzophénone, de préférence en une concentration d'utilisation de 0,1 à 0,5 % en poids.

3. Préparation selon l'une quelconque des revendications précédentes, **caractérisée en ce que** l'agent filmogène non ionique est la PVP, de préférence en une concentration d'utilisation de 0,1 à 3 % en poids.

4. Préparation selon l'une quelconque des revendications précédentes, **caractérisée en ce que** l'oryzanol est contenu en une concentration d'utilisation de 0,01 à 1 % en poids.

5. Préparation selon l'une quelconque des revendications précédentes, **caractérisée en ce qu'**un conditionneur est également contenu.

6. Préparation selon l'une quelconque des revendications précédentes, **caractérisée en ce que** du polyquaternium-10, du polyquaternium-7 ou des composés quaternaires de guar sont utilisés en tant que conditionneur.

7. Préparation selon l'une quelconque des revendications précédentes, **caractérisée en ce qu'**un tensioactif est également contenu.

8. Préparation selon l'une quelconque des revendications précédentes, **caractérisée en ce que** la combinaison de lauryléthersulfate avec de la cocamidopropylbétaïne et du sulfosuccinate est utilisée en tant que tensioactif.

9. Préparation selon l'une quelconque des revendications précédentes, **caractérisée en ce que** la combinaison de lauryléthersulfate avec de la cocamidopropylbétaïne et du décylglucoside est utilisée en tant que tensioactif.

10. Préparation selon l'une quelconque des revendications précédentes, **caractérisée en ce que** le rapport en masse entre le conditionneur et le tensioactif est choisi entre 0,001 et 1.
